(19) Europäisches Patentamt
European Patent Office
Office européen des brevets

(11) Veröffentlichungsnummer: **0 028 375**
**B1**

(12) # EUROPÄISCHE PATENTSCHRIFT

(45) Veröffentlichungstag der Patentschrift: **08.12.82**

(51) Int. Cl.³: **C 07 C 59/52,** C 07 C 57/32, C 07 C 51/377

(21) Anmeldenummer: **80106519.4**

(22) Anmeldetag: **24.10.80**

(54) **Verfahren zur Herstellung von Phenylessigsäure und von deren einfachen Derivaten.**

(30) Priorität: **03.11.79 DE 2944480**

(43) Veröffentlichungstag der Anmeldung: **13.05.81 Patentblatt 81/19**

(45) Bekanntmachung des Hinweises auf die Patenterteilung: **08.12.82 Patentblatt 82/49**

(84) Benannte Vertragsstaaten: **AT BE CH DE FR GB IT LI NL**

(56) Entgegenhaltungen: **EP-A-0 003 825**

(73) Patentinhaber: **HOECHST AKTIENGESELLSCHAFT, Postfach 80 03 20, D-6230 Frankfurt/Main 80 (DE)**

(72) Erfinder: **Spielmann, Werner, Dr., Johann-Strauss-Strasse 18a, D-6233 Kelkheim (Taunus) (DE)**
Erfinder: **Schaeffer, Georg, Dr., Herderstrasse 58, D-6238 Hofheim am Taunus (DE)**

**EP 0 028 375 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Phenylessigsäure und von deren einfachen Derivaten

Phenylessigsäure und deren einfache Derivate wie z. B. o-, m- und p-Hydroxy-, -Alkoxy- und -Halogen-phenylessigsäuren sowie deren Salze und Ester sind wertvolle Zwischen- und teilweise auch Endprodukte auf verschiedenen Gebieten, insbesondere auf dem Pharmagebiet. So besitzt beispielsweise die 3-Fluor-4-hydroxy-phenylessigsäure thyreostatische und 3-Chlor-4-(prop-2-enyloxy)-phenylessigsäure antipyretische Wirksamkeit, das 4-(2'-Hydroxy-3'-isopropylamino-propoxy)-phenylacetamid ist bekannt als $\beta$-Rezeptoren-Blocker.

Für die Herstellung der Phenylessigsäure und von deren Derivaten ist eine Reihe verschiedener Methoden bekannt. Eine gängige Methode zur Herstellung der (unsubstituierten) Phenylessigsäure geht aus von Toluol, das dann zum Benzylchlorid chloriert wird; das durch dessen Umsetzung mit Natriumcyanid erhältliche Benzylcyanid wird dann zur Phenylessigsäure verseift.

Eine übliche Methode zur Herstellung der Säure bedient sich auch der Reduktion von Mandelsäure mit Kaliumiodid, rotem Phosphor und Phosphorsäure, wobei Ausbeuten bis zu 90% d. Th. angegeben werden [Fieser & Fieser, Lehrbuch der organischen Chemie, Verlag Chemie GmbH, Weinheim/Bergstraße, Seite 784/85 (1960)].

Die beiden genannten Methoden sind insbesondere bei der Durchführung in technischem Maßstab nicht unproblematisch (unangenehm zu handhabendes NaCN, teures KJ bzw. HJ).

Man hat auch schon versucht, die Reduktion der Mandelsäure und einiger von deren Derivaten (o-Chlor-, o-Hydroxy-, p-Methoxy-mandelsäure) zu den entsprechenden Phenylessigsäuren katalytisch mit Wasserstoff an einem Pd/$BaSO_4$-Katalysator durchzuführen; die Reduktion gelang zuerst nur bei den entsprechenden O-Acyl-Mandelsäuren, also erst nach Acylierung der aliphatischen OH-Gruppe [Rosenmund und Schindler, Arch. Pharm. 266, 281 (1928)]. Als Lösungsmittel für die Reduktion wurden Xylol, Cumol und Tetralin angewandt, und die mitgeteilten Reaktionstemperaturen betragen bis über 200°C, die Ausbeuten an den Phenylessigsäuren bis 60% d. Th. Die nicht O-acylierten Mandelsäuren wurden unter diesen Bedingungen nicht hydriert.

Die katalytische Hydrierung der Mandelsäure und einiger ihrer Derivate zu den entsprechenden Phenylessigsäuren gelang dann später auch ohne vorherige O-Acylierung bei Raumtemperatur in Eisessig, wenn man dem Eisessig noch bestimmte saure Stoffe (HBr, $ZnCl_2$/HCl, $H_2SO_4$, $HClO_4$) zusetzte [Weidlich, Meyer-Delius, Berichte der Deutschen Chemischen Gesellschaft 73 325—27 (1940); Kindler et. al., Liebigs Annalen 545, 9—15 (1943)]. Die mitgeteilten Ausbeuten an Phenylessigsäure bzw. Phenylessigsäurederivaten betragen bis zu etwa 90% d. Th. Für das Gelingen der Reaktion unter den genannten relativ milden Bedingungen — bei höherer Temperatur erfolgt in zunehmenden Maß auch Kernhydrierung — wird hauptsächlich die Bildung bestimmter Molekülverbindungen der Mandelsäure-(Derivate) mit den dem Eisessig beigefügten sauren Zusatzstoffen sowie auch eine teilweise Veresterung der alkoholischen OH-Gruppe verantwortlich gemacht, wodurch eine gewisse »Aktivierung« dieser alkoholischen OH-Gruppe erfolgen soll. Diese Aktivierung — und damit das gewünschte Hydrierergebnis (Ausbeute an Phenylessigsäure und deren Derivaten) — wird um so schlechter, je ungünstiger die Bedingungen für die Existenz dieser Molekülverbindungen und Ester sind; solche ungünstigen Bedingungen sollen z. B. durch die Anwesenheit von Wasser geschaffen werden [Liebigs Annalen 545, 10 (1943)].

Es ist jedoch auch bekannt geworden, die katalytische Hydrierung der Mandelsäure zur Phenylessigsäure mit guter Ausbeute sogar in wäßriger Lösung durchzuführen — allerdings nicht in reiner wäßriger Lösung, sondern in wäßriger Mineralsäure, insbesondere wäßriger Salzsäure (BE-PS 867 289). Im Falle der Verwendung eines Pd-Katalysators (auf Kohle als Trägermaterial) wird von einer Ausbeute an Phenylessigsäure von 85% d. Th. berichtet. Möglicherweise verläuft die Reaktion also auch hier über eine (durch die Mineralsäure) »aktivierte« Mandelsäure; außerdem soll insbesondere durch die Anwesenheit von Chlorionen eine unerwünschte Kernhydrierung vermieden werden.

Für die bekannten Verfahren zur Herstellung von Phenylesssigsäure (-derivaten) durch katalytische Hydrierung von Mandelsäure (-derivaten) ist nachteilig, daß die Mandelsäure (-derivate) zunächst mehr oder weniger »aktiviert« werden muß bzw. müssen, was mit zusätzlichen Synthesestufen (O-Acylierung) oder mit kostenintensiven Aufarbeitungsoperationen bzw. starken Abwasserbelastungen und Korrosionsproblemen (Eisessig/anorganische Säuren bzw. Salze, wäßrige Mineralsäuren als Lösungsmittel) verbunden ist.

Wegen des ansonsten günstigen Syntheseweges zu Phenylessigsäure und Phenylessigsäurederivate durch katalytische Hydrierung der Mandelsäure und deren entsprechender Derivate war es daher wünschenswert und bestand die Aufgabe, die bekannten katalytischen Reduktionsverfahren zu verbessern.

Diese Aufgabe konnte erfindungsgemäß dadurch gelöst werden, daß man die katalytische Hydrierung in einer wäßrigen Lösung durchführt, welche frei von Mineralsäure, insbesondere von Salzsäure, ist.

Erfindungsgegenstand ist daher ein Verfahren zur katalytischen Hydrierung von Mandelsäure und deren am Kern durch unter den Reaktionsbedingungen im wesentlichen inerte Substituenten ein- oder mehrfach substituierten Derivaten sowie von deren Salzen und Estern an der Carbonsäuregruppe in

Gegenwart eines Edelmetallkatalysators in einer mindestens etwa 50 Gew.-% Wasser enthaltenden wäßrigen Lösung zu Phenylessigsäure und deren entsprechenden Derivaten, dadurch gekennzeichnet ist, daß die wäßrige Lösung frei von Mineralsäure, insbesondere von Salzsäure, ist. Es war aufgrund des einschlägigen Standes der Technik außerordentlich überraschend, daß die katalytische Hydrierung von Mandelsäure (-derivaten) zu Phenylessigsäure (-derivaten) in wäßriger Lösung ohne besondere Zusatzstoffe — also ohne »Aktivierung« der Mandelsäure — in praktisch gleicher Weise und mit praktisch gleichen Ausbeuten verläuft, wie die bekannten, über »aktivierte« Mandelsäure(n) ablaufenden Hydrierungen; denn nach dem besagten Stand der Technik war ohne eine besondere »Aktivierung« der Mandelsäure (-derivate) deren katalytische Reduktion zu Phenylessigsäure (-derivaten) nicht in einer einigermaßen gangbaren Weise zu erwarten. Es war weiterhin überraschend, daß in Abwesenheit von Chlorionen nach dem erfindungsgemäßen Verfahren keine in irgendeiner Weise ins Gewicht fallende Kernhydrierung stattfindet, da nach der BE-PS 867 289 gerade die Anwesenheit von Chlorionen zur Vermeidung einer (unerwünschten) Kernhydrierung notwendig sein sollte.

Als Ausgangsprodukte für das erfindungsgemäße Verfahren kommen Mandelsäure und alle möglichen von deren am Kern (durch unter den Reduktionsbedingungen im wesentlichen inerte Substituenten) ein- oder mehrfach substituierte Derivate sowie deren Salze und Ester in Frage. Bevorzugte Ausgangsprodukte sind die unter die folgende Formel fallenden Verbindungen:

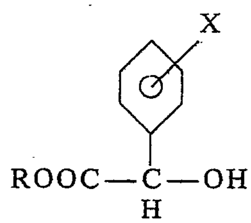

worin

X = OH,
Alkoxy, vorzugsweise $C_1-C_4$-Alkoxy,
Aryloxy, vorzugsweise Phenoxy,
Alkyl, vorzugsweise $C_1-C_4$-Alkyl,
Halogen, vorzugsweise F,
$CF_3$,
$COOR^1$ ($R^1$ = Alkyl, vorzugsweise $C_1-C_4$-Alkyl),
$CONR^2R^3$ ($R^2$, $R^3$ = H, Alkyl, vorzugsweise $C_1-C_4$-Alkyl),
$NR^2R^3$ ($R^2$, $R^3$ = H, Alkyl, vorzugsweise $C_1-C_4$-Alkyl)

R = H,
Me (= Metallkation, vorzugsweise Alkali- oder
$NH_4$-Kation, insbesondere $Na^{\oplus}$),
Alkyl, vorzugsweise $C_1-C_4$-Alkyl,
Aryl, vorzugsweise $C_6H_5$.

Konkrete Ausgangsverbindungen — und zwar solche, welche nicht unter die Formel für die bevorzugten Ausgangsverbindungen fallen als auch solche, welche unter diese Formel fallen — sind beispielsweise:

3-Fluor-4-hydroxymandelsäure
4-Hydroxy-3-ethoxymandelsäure
3-Carboxy-4-hydroxymandelsäure
p-Hydroxymandelsäure
p-Hydroxymandelsäureethylester
p-Methoxymandelsäure
o-Methylmandelsäure
p-tert.-Butylmandelsäure
p-Carbethoxymandelsäureethylester

Besonders bevorzugte Ausgangsprodukte sind m- und p-Hydroxy sowie m- und p-Alkoxy (vorzugsweise $C_1-C_4$-Alkoxy)-Mandelsäure, vor allem die erstgenannten Verbindungen (m- und p-Hydroxymandelsäure), sowie deren Na-Salze.

Als Edelmetallkatalysatoren können für das erfindungsgemäße Verfahren praktisch alle für die Spaltung von Benzyl-O-Bindungen bekannten Katalysatoren verwendet werden [s. Organic Reactions Vol. VII, chapter 5, S. 264 ff. (1953)]. Im einzelnen sind als geeignete Hydrierkatalysatoren hier die

Metalle der 8. Nebengruppe des Periodensystems der Elemente zu nennen (Ru, Rh, Pd, Os, Ir, Pt), wobei Palladium Pd bevorzugt ist. Der Metallkatalysator wird normalerweise auf einem bekannten Trägermaterial wie zum Beispiel auf $SiO_2$ Kohle, Aluminiumoxid, Alumosilikaten, $BaSO_4$ etc., vorzugsweise auf $BaSO_4$ oder $SiO_2$, aufgebracht. Die Konzentration des Metalls auf dem Trägermaterial ist in weiten Grenzen variierbar; vorzugsweise beträgt sie jedoch etwa 1–5 Gew.-%. Bezogen auf das Ausgangsprodukt für das erfindungsgemäße Verfahren ist die Katalysatormenge ebenfalls im Prinzip in weiten Grenzen variierbar; vorteilhaft beträgt die Metallmenge etwa 0,01 bis 0,5 Gew.-%.

Das wesentliche Merkmal des erfindungsgemäßen Verfahrens ist die Durchführung der katalytischen Hydrierung der Mandelsäure und deren entsprechender Derivate in einer von Mineralsäure, insbesondere von Salzsäure freien wäßrigen Lösung. Das dieser Lösung zugrundeliegende Lösungsmittel soll zumindestens 50 (Gew.)% aus Wasser bestehen. Den Rest – wenn das Lösungsmittel nicht zu 100% aus Wasser besteht – können hauptsächlich niedrigsiedende Alkohole, vorzugsweise die $C_1-C_4$-Alkohole (Methanol, Ethanol, n- und i-Propanol (von den Butanolen praktisch nur n- und i-Butanol), gegebenenfalls (bis zu etwa 10%, bezogen auf die Gesamtlösung) auch andere lösungsvermittelnde organische Lösungsmittel wie z. B. Essigsäure, Dioxan etc. ausmachen. Die Alkohole und gegebenenfalls die erwähnten anderen lösungsvermittelnden organischen Lösungsmittel können sowohl einzeln als auch in praktisch beliebigen Mischungen verwendet werden. Bevorzugtes Lösungsmittel für das Verfahren ist jedoch reines Wasser.

Die Konzentration des Ausgangsprodukts in dem Lösungsmittel kann praktisch beliebig gewählt werden, wie bei katalytischen Reduktionen in Lösungen üblich; dabei sind lediglich die Lösungsverhältnisse so wie wirtschaftliche Faktoren (zu verdünnte Lösungen werden unwirtschaftlich!) zu bedenken.

Die Reduktion kann bei Temperaturen praktisch von Raumtemperatur bis etwa 200°C durchgeführt werden; bevorzugt sind jedoch Temperaturen im Bereich von 50 bis 200°C, insbesondere von etwa 90 bis 150°C.

Weiterhin ist es möglich, die Reaktion sowohl bei Normaldruck als auch bei einem Wasserstoffüberdruck bis zu etwa 20 bar, vorzugsweise bis etwa 8 bar durchzuführen. Höhere Drucke bringen im allgemeinen keine Vorteile.

Wenn ohne Wasserstoffüberdruck gearbeitet wird, leitet man zweckmäßig den Wasserstoff durch die bei der Reaktionstemperatur gut gerührte Mischung, wobei überschüssiger Wasserstoff wieder in das Reaktionsgefäß zurückgeführt werden kann.

Bei Anwendung von Wasserstoffüberdruck wird die Hydrierung in einem mit Wasserstoff vorbegasten Druckgefäß durchgeführt.

Eine bevorzugte Variante des erfindungsgemäßen Verfahrens besteht darin, daß man das Ausgangsprodukt während der Hydrierung in das zusammen mit dem Katalysator vorgelegte Lösungsmittel eindosiert; dies gilt sowohl für die Hydrierung ohne als auch mit Wasserstoffüberdruck.

Die Reaktionszeit hängt von den Reaktionsbedingungen (Druck, Temperatur, Konzentrationen) ab und liegt im allgemeinen zwischen etwa 2 und 30 Stunden.

Nach dem Verfahren entsteht aus Mandelsäure Phenylessigsäure und aus den jeweiligen Mandelsäurederivaten die entsprechenden Phenylessigsäurederivate. Die Aufarbeitung und Isolierung der Endprodukte geschieht wie für die Aufarbeitung der Ansätze katalytischer Hydrierungen üblich; eine zweckmäßige Aufarbeitungsmethode besteht darin, daß man den Katalysator heiß abfiltriert und dann das Filtrat gegebenenfalls eindampft. Beim Abkühlen auf Raumtemperatur kristallisiert das Reaktionsprodukt im allgemeinen aus und wird ebenfalls abfiltriert.

Die Ausbeuten an Phenylessigsäure und deren entsprechenden Derivaten liegen in der Größenordnung der auch nach den bekannten Verfahren der katalytischen Reduktion von Mandelsäure (Derivaten) erzielten Ausbeuten (bis zu etwa 90% d. Th.). Die hohen Ausbeuten sowie die Tatsache, daß für das erfindungsgemäße Verfahren keinerlei »aktivierende« Zusatzstoffe verwendet werden, wodurch auch die den bekannten einschlägigen Verfahren anhaftenden Abwasser-, Korrosions- etc. -probleme beim erfindungsgemäßen Verfahren nicht auftreten, stellen einen erheblichen Fortschritt dar.

Das erfindungsgemäße Verfahren wird nun anhand der folgenden Beispiele näher erläutert.

## Beispiel 1

In einer Lösung aus 20 g p-Hydroxymandelsäurehydrat oder der entsprechenden Menge an wasserfreier Säure in 100 ml Wasser werden 0,5 g $Pd/BaSO_4$ (1%ige Belegung) suspendiert. Unter kräftigem Rühren und Durchleiten eines $H_2$-Stromes von ca. 100 l/h, heizt man auf 90°C Innentemperatur. Dabei wird der mit dem Gas ausgetragene Wasserdampf kondensiert und in das Reaktionsgefäß zurückgeführt.

Nach 20 h beträgt der Umsatz nach dünnschichtchromatographischer Kontrolle ca. 80%.

Der Katalysator wird heiß abfiltriert und das erkaltete Filtrat mit Ether extrahiert, um eine Abtrennung der p-Hydroxyphenylessigsäure von umgesetzter p-Hydroxymandelsäure zu erreichen. Nach Eindampfen der Etherlösung erhält man 12,0 g (=75% d. Th.) p-Hydroxyphenylessigsäure.

## Beispiel 2

Eine Mischung aus 20 g p-Hydroxymandelsäurehydrat, 50 ml Wasser und 0,5 g Katalysator (1% Pd/$BaSO_4$) wird in einem Rührautoklaven bei 150°C und einem $H_2$-Partialdruck von 5 bar 3 h hydriert. Anschließend filtriert man den Katalysator heiß ab und läßt das Filtrat über Nacht bei Raumtemperatur stehen, wobei die Hauptmenge der p-Hydroxyphenylessigsäure auskristallisiert. Weiteres Einengen der Mutterlauge liefert weiteres Produkt.

(Gesamt-)Ausbeute: 12,8 g = 80% d. Th.

Das Beispiel wurde unter ansonsten identischen Bedingungen, jedoch mit anderen Katalysatoren wiederholt; die Ergebnisse sind aus folgender Zusammenstellung ersichtlich:

| Katalysator | Ausbeute |
|---|---|
| 1 g 0,5% Pd/$SiO_2$ | 12,4 g = 72% d. Th. |
| 0,1 g 5% Pt/C | 9,2 g = 57% d. Th. |

## Beispiel 3

Eine Suspension von 0,5 g Pd/$BaSO_4$ (1%ige Belegung) in 50 ml Wasser wird in einem Rührautoklaven unter einem $H_2$-Partialdruck von 5 bar auf 150°C erhitzt und eine Lösung von 20 g p-Hydroxymandelsäure-Hydrat in 50 ml Wasser innerhalb von 2 h eingepumpt. Man hydriert noch 2 h nach, filtriert den Katalysator heiß ab, und engt auf ca. 60 ml ein. Beim Abkühlen auf Raumtemperatur kristallisiert über Nacht die Hauptmenge der p-Hydroxyphenylessigsäure aus. Weiteres Produkt wird durch Einengen der Mutterlauge gewonnen.

(Gesamt-)Ausbeute: 13,6 g = 85% d. Th.

## Beispiel 4

In einem Rührautoklaven werden 50 g p-Hydroxymandelsäure-Na-Salz-Hydrat und 5 g 5% Pd/$BaSO_4$ in 300 ml Wasser unter einem $H_2$-Druck von 5 bar auf 115°C aufgeheizt und 10 Stunden bei 115°C hydriert. Man filtriert den Katalysator von der erhaltenen Reaktionslösung ab und säuert mit konz. Salzsäure auf pH 2 an. Die wäßrige Lösung wurde viermal mit je 80 ml Diethylether extrahiert, die vereinigten Etherphasen über $Na_2SO_4$ getrocknet und zur Trockne eingedampft. Man erhielt 18,6 g p-Hydroxyphenylessigsäure (= 85% d. Th., bezogen auf umgesetztes p-Hydroxymandelsäure-Na-Salz-Hydrat).

Unumgesetzte p-Hydroxymandelsäure kann in Form des Na-Salzes aus der wäßrigen Phase zurückgewonnen werden. Dazu stellt man mit 2 n NaOH auf pH 6,5 ein und dampft im Vakuum auf ein geringes Volumen ein. Aus dem erhaltenen Rückstand kristallisieren ca. 20 g p-Hydroxymandelsäure-Na-Salz-Hydrat aus.

**Patentansprüche**

1. Verfahren zur katalytischen Hydrierung von Mandelsäure und deren am Kern durch unter den Reaktionsbedingungen im wesentlichen inerte Substituenten ein- oder mehrfach substituierten Derivaten sowie von deren Salzen und Estern an der Carbonsäuregruppe in Gegenwart eines Edelmetallkatalysators in einer mindestens 50 Gew.-% Wasser enthaltenden wäßrigen Lösung zu Phenylessigsäure und deren entsprechenden Derivaten, dadurch gekennzeichnet, daß die wäßrige Lösung frei von Mineralsäure, insbesondere von Salzsäure, ist.

2. Verfahren nach Anspruch 1, dadurch gekennzeichnet, daß man als Ausgangsprodukte Verbindungen der folgenden Formel verwendet:

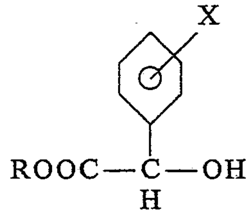

worin

X = OH,
Alkoxy, vorzugsweise $C_1-C_4$-Alkoxy,
Aryloxy, vorzugsweise Phenoxy,
Alkyl, vorzugsweise $C_1-C_4$-Alkyl,
Halogen, vorzugsweise F,
$CF_3$,
$COOR^1$ ($R^1$ = Alkyl, vorzugsweise $C_1-C_4$-Alkyl),
$CONR^2R^3$ ($R^2$, $R^3$ = H, Alkyl, vorzugsweise $C_1-C_4$-Alkyl),
$NR^2R^3$ ($R^2$, $R^3$ = H, Alkyl, vorzugsweise $C_1-C_4$-Alkyl),
R = H,
Me (= Metallkation vorzugsweise Alkali- oder $NH_4$-Kation, insbesondere $Na^{\oplus}$),
Alkyl, vorzugsweise $C_1-C_4$-Alkyl,
Aryl, vorzugsweise $C_6H_5$.

3. Verfahren nach Ansprüchen 1 bis 2, dadurch gekennzeichnet, daß man als Ausgangsprodukte m- und p-Hydroxy oder m- und p-Alkoxy-Mandelsäure, vorzugsweise m- und p-Hydroxy-Mandelsäure sowie deren Na-Salze verwendet.

4. Verfahren nach Ansprüchen 1 bis 3, dadurch gekennzeichnet, daß man als Edelmetallkatalysator Palladium auf Bariumsulfat oder $SiO_2$ als Träger verwendet.

5. Verfahren nach Ansprüchen 1 bis 4, dadurch gekennzeichnet, daß man die katalytische Hydrierung bei Temperaturen von 50 bis 200° C, vorzugsweise von etwa 90 bis etwa 150° C, durchführt.

6. Verfahren nach Ansprüchen 1 bis 5, dadurch gekennzeichnet, daß man das Ausgangsprodukt während der Hydrierung in das zusammen mit dem Katalysator vorgelegte Lösungsmittel eindosiert.

**Claims**

1. Process for the catalytic hydrogenation of mandelic acid and the derivatives thereof mono- or polysubstituted at the nucleus by substituents substantially inert under the reaction conditions, as well as of the salts and esters at the carboxylic acid group thereof in the presence of a noble metal catalyst in aqueous solution containing at least 50% by weight water, characterized in the aqueous solution being free from mineral acid, especially free from hydrochloric acid.

2. The process of claim 1, characterized in using as starting products compounds of the following formula

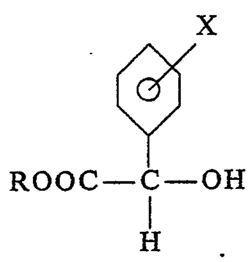

in which

X denotes OH,
alkoxy, preferably $C_1-C_4$ alkoxy,
aryloxy, preferably phenoxy,
alkyl, preferably $C_1-C_4$ alkyl,
halogen preferably F,
$CF_3$,
$COOR^1$ with $R^1$ being alkyl, preferably $C_1-C_4$ alkyl,
$CONR^2R^3$ with $R^2$ and $R^3$ being H, alkyl, preferably $C_1-C_4$ alkyl,
$NR^2R^3$ with $R^2$ and $R^3$ being H, alkyl, preferably $C_1-C_4$ alkyl,
R denotes H,
M (a metal cation, preferably an alkali metal or $NH_4$ cation, more preferably $Na^{\oplus}$),
alkyl, preferably $C_1-C_4$ alkyl,
or aryl, preferably $C_6H_5$.

3. The process of claims 1 to 2, characterized in the starting product being m- or p-hydroxymandelic acid or m- or p-alkoxymandelic acid, preferably m- or p-hydroxymandelic acid, and the sodium salts thereof.

4. The process of claims 1 to 3, characterized in using as catalyst palladium on barium sulfate or $SiO_2$ as carrier.

5. The process of claims 1 to 4, characterized in carrying out the catalytic hydrogenation at temperatures of from 50° C to 200° C, preferably of from about 90° C to about 150° C.

6. The process of claims 1 to 5, characterized in adding during hydrogenation the starting product to the solvent containing the catalyst.

**Revendications**

1. Procédé pour hydrogéner catalytiquement l'acide mandélique et des acides mandéliques portant, sur le noyau, un ou plusieurs substituants pratiquement inertes dans les conditions de la réaction, ainsi que leurs sels et esters au niveau du groupe carboxy, en présence d'un catalyseur à base d'un métal noble, dans une solution aqueuse contenant au moins 50% en poids d'eau, et les convertir ainsi en l'acide phénylacétique et en les dérivés correspondants, procédé caractérisé en ce que la solution aqueuse est dépourvue d'acide minéral, en particulier d'acide chlorhydrique.

2. Procédé selon la revendication 1 caractérisé en ce qu'on utilise, comme corps de départ, des composés répondant à la formule:

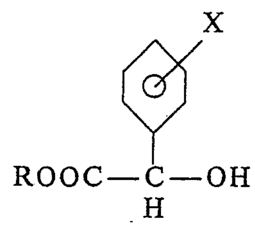

dans laquelle

X représente $-OH$, un radical alcoxy, de préférence en $C_1-C_4$, un radical aryloxy, de préférence phénoxy, un radical alkyle, de préférence en $C_1-C_4$, un halogène, de préférence F, le radical $-CF_3$, un radical $-COOR^1$ dans lequel $R^1$ désigne un alkyle, de préférence un alkyle en $C_1-C_4$, un radical $-CONR^2R^3$ dans lequel $R^2$ et $R^3$ représentent chacun l'hydrogène ou un alkyle, de préférence un alkyle en $C_1-C_4$, ou X représente un radical $-NR^2R^3$ dans lequel $R^2$ et $R^3$ représentent chacun l'hydrogène ou un alkyle, de préférence un alkyle en $C_1-C_4$, et

R représente l'hydrogène, Me (cation métallique, de préférence cation de métal alcalin ou cation $NH_4$, plus particulièrement $Na^{\oplus}$, un radical alkyle, de préférence en $C_1-C_4$, ou un radical aryle, de préférence $C_6H_5$.

3. Procédé selon l'une des revendications 1 et 2, caractérisé en ce qu'on utilise, comme corps de départ, l'acide m- ou p-hydroxy-mandélique ou un acide m- ou p-alcoxy-mandélique, de préférence l'acide m- ou p-hydroxy-mandélique, ou leurs sels sodiques.

4. Procédé selon l'une quelconque des revendications 1 à 3, caractérisé en ce qu'on utilise, comme catalyseur à base d'un métal noble, du palladium déposé sur sulfate de baryum ou sur $SiO_2$.

5. Procédé selon l'une quelconque des revendications 1 à 4, caractérisé en ce qu'on effectue l'hydrogénation catalytique à des températures de 50 à 200° C, de préférence d'environ 90 à environ 150° C.

6. Procédé selon l'une quelconque des revendications 1 à 5, caractérisé en ce qu'on introduit progressivement le corps de départ, au cours de l'hydrogénation, dans le solvant préalablement mis en place avec le catalyseur.